# EUROPÄISCHE PATENTSCHRIFT

(11) **EP 1 455 188 B1**
(45) Veröffentlichungstag und Bekanntmachung des Hinweises auf die Patenterteilung: **25.07.2007**
(21) Anmeldenummer: 04005068.4
(22) Anmeldetag: 04.03.2004
(51) Int. Cl.: G01N 33/543

(54) **Verfahren zur Immobilisierung von Glykoproteinen**
Method for immobilising glycoproteins
Procédé pour l'immobilisation des glycoprotéines

(30) Priorität: 06.03.2003 DE 10310193
(43) Veröffentlichungstag der Anmeldung: 08.09.2004
(73) Patentinhaber: Micronas Holding GmbH, 79108 Freiburg (DE); Klapproth, Holger, Dr., 79108 Freiburg (DE)
(72) Erfinder: Klapproth, Holger, 79108 Freiburg (DE)
(74) Vertreter: Stürken, Joachim

(56) Entgegenhaltungen:
- WO-A-02/057422
- US-A- 4 874 813
- US-A- 5 543 054
- US-A- 2002 001 845
- COLLIOUD A ET AL: "ORIENTED AND COVALENT IMMOBILIZATION OF TARGET MOLECULES TO SOLID SUPPORTS: SYNTHESIS AND APPLICATION OF A LIGHT-ACTIVATABLE AND THIOL-REACTIVE CROSS-LINKING REAGENT" BIOCONJUGATE CHEMISTRY, AMERICAN CHEMICAL SOCIETY, WASHINGTON, US, Bd. 4, Nr. 6, 1993, Seiten 528-536, XP001121631 ISSN: 1043-1802
- SCHRIVER-LAKE L C ET AL: "ANTIBODY IMMOBILIZATION USING HETEROBIFUNCTIONAL CROSSLINKERS" BIOSENSORS & BIOELECTRONICS, ELSEVIER SCIENCE PUBLISHERS, BARKING, GB, Bd. 12, Nr. 11, 1997, Seiten 1101-1106, XP000998798 ISSN: 0956-5663
- Wikipedia encyclopedia: definition of copolymer (in German), definition of heteropolymer (in English) as on the internet on 20.06.2006.

## Beschreibung

Die vorliegende Erfindung betrifft allgemein das Gebiet der Biotechnologie. Insbesondere betrifft die Erfindung ein Verfahren zur kovalenten Immobilisierung von Glykoproteinen wie Immunglobulinen auf feste, halbfeste oder gelartige Oberflächen, und dafür geeignete Konstrukte, sowie Oberflächen und Vorrichtungen, welche entsprechend immobilisierte Glykoproteine aufweisen.

Die wissenschaftlichen und kommerziellen Anwendungsgebiete für Oberflächen mit funktional zugänglichen Glykoproteinen oder funktionellen Fragmenten derselben sind vielfältig und erfordern angesichts steigender Leistungs- und Qualitätsanforderungen an damit ausgestattete Vorrichtungen eine ständige Optimierung der zugrunde liegenden Basistechnologie.

Bei der Immobilisierung von Proteinen an im wesentlichen feste Oberflächen wird grundsätzlich zwischen kovalenter und nichtkovalenter Kopplung unterschieden. Während die Adsorption (Anlagerung) von Molekülen an eine Oberfläche (z.B. aus Polystyrol) aufgrund intermolekularer Anziehungskräfte (van der Waals'sche Kräfte) erfolgt, sind die "eigentlichen" chemischen Bindungen durch Ausbildung gemeinsamer Elektronenpaare (kovalent) oder stöchiometrischer Ladungsunterschiede (ionisch) gekennzeichnet.

Für die Immobilisierung von Immunglobulinen wie spezifischen Antikörpern werden zumeist Bedingungen geschaffen, unter denen eine Bindung durch (hydrophobe) Wechselwirkungen stattfinden kann. Alternativ werden Verfahren unter Verwendung geeigneter Vernetzungsmittel (Crosslinker) insbesondere dann angewendet, wenn für den gewünschten Anwendungszweck die Vorteile einer dauerhaften kovalenten Kopplung realisiert werden sollen. Derartige Verfahren sowie geeignete Crosslinker sind dem Fachmann bekannt und gelten als etabliert (z.B. G. Hermannson, Bioconjugate Techniques, Academic Press (1996)).

Die US 2002/0001845 A1 betrifft beispielsweise die Immobilisierung von Molekülen auf Oberflächen unter Verwendung polymerer Strukturen, an deren funktionelle Gruppen diese Moleküle kovalent gebunden werden können. Die Polymer-Strukturen können sowohl in Wasser lösliche bzw. in Wasser dispergierbare Segmente als auch andere Segmente umfassen, die gegenüber der Substratoberfläche eine gewisse Affinität aufweisen und eine Verankerung durch hydrophobe Wechselwirkung, Wasserstoffbrücken oder Coulombsche Kräfte ermöglichen sollen.

Die im Stand der Technik bekannten Crosslinker-Verfahren weisen z.T. gravierende Nachteile auf, durch welche die Qualität entsprechend hergestellter Oberflächen und Vorrichtungen hinsichtlich Spezifität und Sensitivität negativ beeinträchtigt wird. Beispielsweise erfolgt die kovalente Verknüpfung zwischen Crosslinker und Protein über aktivierte Kopplungsgruppen, die z.B. für nachfolgende, auf der spezifischen Bindung zwischen Protein (Rezeptor) und Bindungspartner (Ligand) basierende Untersuchungen nicht mehr zur Verfügung stehen. Ferner lassen es die bekannten Verfahren aufgrund der fehlenden Selektivität der aktivierten Kopplungsgruppen zu, dass mehrere Crosslinker-Moleküle am Protein gebunden sind, und dass entsprechend hergestellte Konstrukte mit ihresgleichen reagieren und daher an nachfolgenden Analysen nicht beteiligt werden können. Ein weiterer Nachteil besteht darin, dass das Protein über seine aktivierten Kopplungsgruppen direkt an die Oberfläche bindet und anschließende Untersuchungen aufgrund sterischer Probleme scheitern. Im Zusammenhang mit Glykoproteinen wie insbesondere Antikörpern bedeuten die Nachteile, dass die für eine spezifische Rezeptor/Ligand-Interaktion erforderlichen Bindungsdomänen oder Epitope des Proteins nicht oder lediglich in nicht reproduzierbarer Weise zur Verfügung stehen.

Die Aufgabe der vorliegenden Erfindung liegt daher in der Schaffung alternativer Konstrukte und Verfahren zur Immobilisierung derselben, mit denen die Nachteile des Standes der Technik überwunden werden.

Die Aufgabe wird erfindungsgemäß durch Bereitstellung eines Konstrukts gemäß Hauptanspruch gelöst. Besondere Ausführungsformen des Konstrukts sind in den Unteransprüchen dargestellt. Ferner werden mit dem Konstrukt versehene Oberflächen und Vorrichtungen sowie Herstellungsverfahren und Verwendungen bereitgestellt, die Gegenstand von Nebenansprüchen sind. Besondere Ausführungsformen derselben sind in den jeweiligen Unteransprüchen ausgeführt.

Zahlreiche wichtige Zelloberflächenproteine und sekretorische Proteine weisen eine oder mehrere kovalent gebundene Mono- und/oder Oligosaccharide auf und werden daher als glykosylierte Proteine oder Glykoproteine bezeichnet. Mit Ausnahme von Bakterienzellen sind Glykoproteine in nahezu allen Organismen und Zelltypen weit verbreitet. Als charakteristische Zuckerkomponenten der Glykoproteine gelten die Hexosen Galactose, Mannose und Glucose sowie die N-Acetylhexosamine (N-Acetylgalactosamin, N-Acetylglucosamin) und als Kettenglieder Sialinsäure (N-Acetylneuraminsäure) und Fucose. Die Oligosaccharidketten sind - meist verzweigt und ohne periodische Sequenz - aus im allgemeinen 2 bis 10 Monosaccharideinheiten aufgebaut. Die Bindung der Mono- bzw. Oligosaccharide an die betreffenden Proteinkomponenten ist O-glykosidisch über die Hydroxylgruppen von Serin- bzw. Threonin-Resten, N-glykosidisch über terminale Aminogruppen, ω-Aminogruppen von Lysin-Resten bzw. Amidgruppe von Asparagin-Resten, und/oder Ester-glykosidisch über die Carboxylgruppen von Asparagin- und Glutaminsäure-Resten. Die Anzahl der Kohlenhydrat-Verknüpfungsstellen bzw. der Kohlenhydratanteil von Glykoproteinen schwankt zwischen nur einer und mehreren hundert. Die Glykoproteine sind wesentliche Bestandteile der Zellmembran sowie der extrazellulären Matrix (Glykosaminglucane, Lectine, Zellwandproteine). Das Glykosylierungsmuster der Glykoproteine hat Informationscharakter und wird daher für einen gewebe- und zelltypspezifischen sowie entwicklungsabhängigen Aufbau der Zelloberfläche benötigt. Das Immunsystem, Viren und Spermien z.B. benötigen diese hochspezifischen Muster zur Erkennung und Adsorption an die Zielzelle. Aufgrund ihrer zahlreichen Hydroxylgruppen erhöhen gebundene Kohlenhydrate oft die Löslichkeit von Proteinen.

Die Strukturen von N- und O-verknüpften Oligosacchariden sind sehr verschieden. Gemeinsames Merkmal aller Glykoproteine ist jedoch das Vorhandensein mindestens eines terminalen Glykosylrestes, der durch Oxidation unter geeigneten Bedingungen in die Aldehydform überführt werden kann.

Die Klasse der Glykoproteine umfasst die Immunglobuline, welche nach Kontakt des Organismus mit einem Antigen von B-Lymphozyten bzw. Plasmazellen gebildet werden und als Antikörper in Serum, Gewebsflüssigkeiten und Körpersekreten für die humorale Immunität bedeutsam sind. Sie bestehen aus zwei jeweils paarweise identischen Polypeptidketten mit einem Kohlenhydratanteil, den sog. Leichten oder L-Ketten und den sog. Schweren oder H-Ketten, die über symmetrisch angeordnete Disulfidbrücken miteinander verbunden sind. Während der aminoterminale, in seiner Aminosäuresequenz variable Teil des Moleküls die Antigenbindungsstellen trägt, weist der carboxyterminale Teil eine relativ konstante Struktur auf. Nach Aufspaltung durch proteolytische Enzyme entstehen verschiedene Immunglobulinfragmente (funktionelle Fragmente); durch Papain zwei identische monovalente Fab-Fragmente (Antigen-bindende Fragmente ohne agglutinierende oder präzipitierende Eigenschaften) und ein Fc-Fragment (für bestimmte biologische Funktionen wie der Bindung an zelluläre Rezeptoren), durch Pepsin ein divalentes sog. F(ab')₂-Fragment (bewirkt nach Antigenbindung eine Agglutination oder Präzipitation) und kleinere Bruchstücke des Fc-Fragments. Bekanntermaßen erfolgt die Einteilung der Immunglobuline nach physikochemischen und biologischen (physiologischen und antigenen) Eigenschaften der schweren Ketten in 5 Immunglobu-linklassen (IgG, IgM, IgA, IgD, IgE). Die leichten Ketten werden aufgrund ihrer Primärstruktur und antigenen Eigenschaften in zwei Typen (κ und λ) unterteilt und sind nicht klassenspezifisch.

Der Kohlenhydratanteil von z.B. Serumantikörperproteinen enthält gewöhnlich N-Acetylglucosamin, Mannose, Fucose, Galactose und N-Acetylneuraminsäure (NANA). Die Unterschiede zwischen verschiedenen Proteinen betreffen hauptsächlich die Anzahl von Verzweigungen, die Anzahl von NANA-Resten, sowie die chemische Natur der Verknüpfung zwischen N-Acetylneuraminsäure und Galactose. Der bei Antikörpern im Bereich der schweren Ketten gebundene Kohlenhydratanteil trägt an seinem äußersten Ende also zumeist einen NANA-Rest, welcher negativ geladen ist und die Löslichkeit des Moleküls steigert.

Von dem Begriff "Glykoprotein" sind erfindungsgemäß nicht nur native Moleküle umfasst, wie sie aus natürlichen Quellen isoliert werden können, sondern auch davon abgeleitete Formen, Fragmente und Derivate, sowie rekombinante Formen und artifizielle Moleküle, sofern sie mindestens eine Eigenschaft der nativen Moleküle aufweisen.

Nach einem ersten Aspekt betrifft die vorliegende Erfindung ein Konstrukt umfassend ein Glykoprotein und ein heterobifunktionelles Crosslinker-Molekül mit Spacer-Bereich, welcher einen Abschnitt zur nicht-kovalenten Anlagerung an eine Oberfläche aufweist, wobei der Spacer-Bereich als Blockcopolymer sowohl einen hydrophilen als auch einen hydrophoben Abschnitt umfasst, und wobei das Crosslinker-Molekül zur kovalenten Verknüpfung mit der Oberfläche mindestens eine photoreaktive Gruppe aufweist.

Erfindungsgemäß hat sich gezeigt, dass die Immobilisierung eines Glykoproteins oder funktionellen Fragments desselben an einer definierten, diskreten Position der Oberfläche durch Bereitstellung eines Abschnittes im Spacer-Bereich zur nichtkovalente Anlagerung signifikant erleichtert wird.

Der dem Glykoprotein zugewandte Teil des Spacers ist hinsichtlich seiner chemischen Eigenschaften mit dem Lösungsmittel bzw. Puffersystem, in welchem die anschließende Interaktion zwischen Rezeptor und Ligand stattfinden soll, vorzugsweise kompatibel. Sofern die Inkubation der zu untersuchenden Probe mit dem immobilisierten Konstrukt in wässrigem Medium erfolgt, kann der Spacer beispielsweise aus Polyethylenglycol oder Polyethylenoxid bestehen. In diesem Fall sollte der vom Glykoprotein wegweisende Teil des Spacers vorzugsweise derart beschaffen sein, dass er nicht mit dem erstgenannten Teil reagieren kann und ferner in der Lage ist, mit der Oberfläche, an die immobilisiert werden soll, zu interagieren.

Nach einer bevorzugten Ausführungsform ist der Abschnitt zur nicht-kovalenten Anlagerung in Abhängigkeit der Eigenschaften der zu beaufschlagenden Oberfläche hydrophob oder unter positiv oder negativ geladenen hydrophilen Abschnitten ausgewählt. In den meisten praktischen Anwendungen werden hydrophobe Oberflächen (z.B. Polystyrol) eingesetzt, so dass der Spacer-Abschnitt überwiegend hydrophobe Eigenschaften aufweisen sollte. Bei Verwendung von hydrophilen Oberflächen sollte der Spacer-Abschnitt hydrophil sein und über Ladungen verfügen, deren Polarität sich von derjenigen der Oberfläche unterscheidet.

Handelt es sich beispielsweise um eine hydrophile, negativ geladene Oberfläche, sollte der Abschnitt des Spacers aus polykationischen Monomeren bestehen. Sofern an eine hydrophile, positiv geladene Oberfläche immobilisiert werden soll, sollte er demgegenüber aus polyanionischen Monomeren aufgebaut sein.

Da die meisten unter Verwendung von Antikörpern oder funktionellen Fragmenten derselben durchgeführten Rezeptor-/Liganden-Assays in wässrigen Lösungsmittel- oder Puffersystemen erfolgen, sollte sich der hydrophile Teil am Glykoprotein befinden, so dass der hydrophobe Teil für die Immobilisierung an die zumeist hydrophobe Oberfläche zur Verfügung steht. Wenn das zur Immobilisierung vorgesehene Konstrukt aus Glykoprotein und Crosslinker-Molekül einschließlich Spacer auf die Oberfläche appliziert wird, erfolgt zunächst eine Adsorption des Konstruktes an die hydrophobe Oberfläche. Da der hydrophobe Abschnitt des Spacers zur Anlagerung an die hydrophobe Oberfläche neigt, erfolgt die kovalente Immobilisierung des Konstrukts im Applikationsbereich über die im hydrophoben Abschnitt befindliche Crosslinkergruppe.

Nach einer bevorzugten Ausführungsform befindet sich die erfindungsgemäß geforderte mindestens eine photoreaktive Gruppe des Crosslinker-Moleküls zur kovalenten Verknüpfung mit der Oberfläche endständig am Spacer und/oder sie ist integraler Bestandteil des Spacers und kann durch Bestrahlung mit Lichtenergie im bevorzugten Wellenlängenbereich von 260 bis 320 nm aktiviert werden (photoreaktive Gruppe).

Die Auswahl geeigneter photoreaktiver Gruppen erfolgt vorzugsweise unter Arylaziden, Benzophenonen, Benzothionen, Anthrachinonen, Anthrathionen, und Thymidin.

Der Einbau von einer oder mehreren erfindungsgemäß geeigneten, auch voneinander verschiedenen photoreaktiven Gruppen kann mittels herkömmlicher Verfahren durch Copolymerisation (z.B. durch DNA-Synthesegeräte) bei der Herstellung des Spacers und/oder mittels gewöhnlicher Crosslinker an das Ende des fertigen Spacers erfolgen. Ein hierfür geeigneter Crosslinker ist z.B. NHS-ASA (Pierce, Product No. 27714).

Ein erfindungsgemäß bevorzugter Spacer kann mit einem üblichen DNA-Synthesegerät hergestellt werden und umfasst einen Amino-Linker (als Phosphoramidit verfügbar), gefolgt von 15 Einheiten Desoxyinosin (hydrophil), 15 C6-Spacern (hydrophob) und 3 Einheiten Desoxythymidin (photoreaktive Gruppen).

Weiterhin ist bevorzugt, dass das Crosslinker-Molekül mit dem Glykoprotein über dessen Kohlenhydratanteil kovalent verknüpft ist.

Die funktionell für die Kopplung des Glykoproteins vorgesehene reaktive Gruppe des Crosslinkers wird in Abhängigkeit der chemischen Eigenschaften der zur Kopplungsreaktion auf Seiten des Glykoproteins zur Verfügung stehenden aktivierbaren Gruppen vorzugsweise unter solchen ausgewählt, die gegenüber Aldehydgruppen reaktiv sind. Ein erfindungsgemäß besonders geeignetes Crosslinker-Molekül verfügt daher über eine Aminogruppe, so dass die Kopplung des Glykoproteins über dessen aktivierte Aldehydgruppe erfolgen kann. Im Falle der gewünschten Immobilisierung z.B. eines Antikörperfragments ist bevorzugt, dass das Crosslinker-Molekül über eine Maleinimidgruppe verfügt, über die das funktionelle Fragment des Glykoproteins über dessen Thiolgruppe(n) kovalent verknüpft werden kann.

Ferner ist besonders bevorzugt, dass das Glykoprotein oder das Fragment ein Antikörper beziehungsweise ein Fab- oder F(ab')₂-Fragment oder ein scab-Protein ist, wobei das Protein oder Fragment rekombinanten Ursprungs sein kann.

Nach einem weiteren Aspekt betrifft die vorliegende Erfindung eine Oberfläche mit einem kovalent daran immobilisierten Konstrukt der zuvor definierten, erfindungsgemäßen Art, wobei eine Oberfläche mit einer Vielzahl erfindungsgemäßer Konstrukte für parallelisierte Untersuchungen bevorzugt ist.

Das Material der erfindungsgemäßen Oberfläche ist vorzugsweise ausgewählt aus der Gruppe bestehend aus Glas, Quarzglas, Quarz, Silizium, Polymeren einschließlich PMMA, Polystyrol, Polyethylen, Polypropylen und PVC, Membranen einschließlich solchen aus Nitrozellulose, Nylon und Mikrofasern, und Papier, sowie Mischformen derselben.

Nach einem weiteren Aspekt betrifft die vorliegende Erfindung eine analytische oder diagnostische Vorrichtung, welche ein erfindungsgemäßes Konstrukt oder eine Oberfläche der zuvor definierten Art umfasst, wobei Vorrichtungen in Form eines Biochips, Sensorchips, Reaktionsröhrchens oder einer Mikrotiterplatte besonders bevorzugt sind.

Aus den obigen Ausführungen folgt, dass die vorliegende Erfindung ferner ein Verfahren betrifft zur Herstellung eines Konstrukts umfassend ein Glykoprotein und ein heterobifunktionelles Crosslinker-Molekül mit Spacer-Bereich, welcher einen Abschnitt zur nicht-kovalenten Anlagerung an eine Oberfläche aufweist, wobei der Spacer-Bereich als Blockcopolymer sowohl einen hydrophilen als auch einen hydrophoben Abschnitt umfasst, und wobei das Crosslinker-Molekül zur kovalenten Verknüpfung mit der Oberfläche mindestens eine photoreaktive Gruppe aufweist, umfassend die folgenden Schritte:
(a) Inkubation des Glykoproteins unter geeigneten Bedingungen zur Schaffung von Aldehyd- beziehungsweise Thiolgruppen;
(b) Kovalente Verknüpfung des Produkts aus Schritt (a) mit dem Crosslinker-Molekül über dessen Amino- beziehungsweise Maleinimidgruppe(n); sowie gegebenenfalls
(c) Aufreinigung und/oder Isolierung des Konstrukts.

Erfindungsgemäß hat sich herausgestellt, dass Glykoproteine wie insbesondere Immunglobuline (Antikörper) in definierter Orientierung auf Oberflächen immobilisiert werden können, indem der mindestens eine Glycosylrest des Kohlenhydratanteils unter oxidativen Bedingungen in ein Aldehyd überführt wird und die anschließende Immobilisierung des modifizierten Moleküls an eine aminomodifizierte Oberfläche erfolgt. Auf diese Weise wird sichergestellt, dass die Bindungsdomänen derart kovalent gebundener Immunglobuline ungehindert für die im Rahmen medizinischer und diagnostischer Anwendungen gewünschten Interaktionen zwischen Rezeptor und Ligand zur Verfügung stehen.

Wie zuvor ausgeführt, weist das heterobifunktionelle Crosslinker-Molekül zusätzlich zur Amino- oder Maleinimidgruppe als zweite funktionelle Gruppe mindestens eine photoreaktive Gruppe auf, und der Spacer-Bereich umfasst einen Abschnitt zur nicht-kovalenten Anlagerung an eine Oberfläche, wobei bezüglich dieser Merkmale auf die obigen Ausführungen verwiesen wird. Es ist bevorzugt, dass sich die mindestens eine photoreaktive Gruppe endständig am Spacer befindet und/oder integraler Bestandteil des Spacers ist.

Ferner betrifft die vorliegende Erfindung ein Verfahren zur kovalenten Immobilisierung eines Glykoproteins auf einer Oberfläche unter Verwendung eines heterobifunktionellen Crosslinker-Moleküls, umfassend die folgenden Schritte:
(a) Aufbringen eines Konstrukts aus einem Glykoprotein und einem heterobifunktionellen Crosslinker-Molekül mit Spacer-Bereich, welcher einen Abschnitt zur nicht-kovalenten Anlagerung an eine Oberfläche aufweist, wobei der Spacer-Bereich als Blockcopolymer sowohl einen hydrophilen als auch einen hydrophoben Abschnitt umfasst, und mindestens einer photoreaktiven Gruppe auf eine Oberfläche; und
(b) Immobilisieren des Konstrukts auf der Oberfläche durch Bestrahlen der Kontaktstelle mit Licht einer geeigneten Wellenlänge.

Schließlich betrifft die vorliegende Erfindung die Verwendung des erfindungsgemäßen Konstrukts, der erfindungsgemäßen Oberfläche, oder der erfindungsgemäßen Vorrichtung im Rahmen analytischer oder diagnostischer Vorrichtungen.

## Patentansprüche

1. Konstrukt umfassend ein Glykoprotein und ein heterobifunktionelles Crosslinker-Molekül mit Spacer-Bereich, welcher einen Abschnitt zur nicht-kovalenten Anlagerung an eine Oberfläche aufweist, wobei der Spacer-Bereich als Blockcopolymer sowohl einen hydrophilen als auch einen hydrophoben Abschnitt umfasst, und wobei das Crosslinker-Molekül zur kovalenten Verknüpfung mit der Oberfläche mindestens eine photoreaktive Gruppe aufweist.

2. Konstrukt nach Anspruch 1, **dadurch gekennzeichnet, dass** der Abschnitt zur nicht-kovalenten Anlagerung hydrophob oder unter positiv oder negativ geladenen hydrophilen Abschnitten ausgewählt ist.

3. Konstrukt nach Anspruch 1 oder 2, **dadurch gekennzeichnet, dass** sich die mindestens eine photoreaktive Gruppe endständig am Spacer befindet und/oder integraler Bestandteil des Spacers ist.

4. Konstrukt nach einem der Ansprüche 1 bis 3, **dadurch gekennzeichnet, dass** die mindestens eine photoreaktive Gruppe ausgewählt ist unter Arylaziden, Benzophenonen, Benzothionen, Anthrachinonen, Anthrathionen, Thymidin, und Derivaten davon.

5. Konstrukt nach einem der vorhergehenden Ansprüche, **dadurch gekennzeichnet, dass** das Crosslinker-Molekül mit dem Glykoprotein über dessen Kohlenhydratanteil kovalent verknüpft ist.

6. Konstrukt nach einem der vorhergehenden Ansprüche, **dadurch gekennzeichnet, dass** das Glykoprotein ein Antikörper ist.

7. Konstrukt nach einem der vorhergehenden Ansprüche, **dadurch gekennzeichnet, dass** das Glykoprotein rekombinanten Ursprungs ist.

8. Oberfläche mit einem kovalent daran immobilisierten Konstrukt gemäß einem der Ansprüche 1 bis 7.

9. Oberfläche nach Anspruch 8, umfassend eine Vielzahl von Konstrukten gemäß einem der Ansprüche 1 bis 7.

10. Oberfläche nach Anspruch 8 oder 9, dessen Material ausgewählt ist aus der Gruppe bestehend aus Glas, Quarzglas, Quarz, Silizium, Polymeren einschließlich PMMA, Polystyrol, Polyethylen, Polypropylen und PVC, Membranen einschließlich solchen aus Nitrozellulose, Nylon und Mikrofasern, und Papier, sowie Mischformen derselben.

11. Analytische oder diagnostische Vorrichtung, umfassend ein Konstrukt gemäß einem der Ansprüche 1 bis 7 oder eine Oberfläche gemäß einem der Ansprüche 8 bis 10.

12. Analytische oder diagnostische Vorrichtung nach Anspruch 11 in Form eines Biochips, Sensorchips, Reaktionsröhrchens oder einer Mikrotiterplatte.

13. Verfahren zur Herstellung eines Konstrukts umfassend ein Glykoprotein und ein heterobifunktionelles Crosslinker-Molekül mit Spacer-Bereich, welcher einen Abschnitt zur nicht-kovalenten Anlagerung an eine Oberfläche aufweist, wobei der Spacer-Bereich als Blockcopolymer sowohl einen hydrophilen als auch einen hydrophoben Abschnitt umfasst, und wobei das Crosslinker-Molekül zur kovalenten Verknüpfung mit der Oberfläche mindestens eine photoreaktive Gruppe aufweist, umfassend die folgenden Schritte:
(a) Inkubation des Glykoproteins unter geeigneten Bedingungen zur Schaffung von Aldehyd- beziehungsweise Thiolgruppen;
(b) Kovalente Verknüpfung des Produkts aus Schritt (a) mit dem Crosslinker-Molekül über dessen Amino- beziehungsweise Maleinimidgruppe(n); sowie gegebenenfalls
(c) Aufreinigung und/oder Isolierung des Konstrukts.

14. Verfahren nach Anspruch 13, **dadurch gekennzeichnet, dass** sich die mindestens eine photoreaktive Gruppe endständig am Spacer befindet und/oder integraler Bestandteil des Spacers ist.

15. Verfahren zur kovalenten Immobilisierung eines Glykoproteins auf einer Oberfläche unter Verwendung eines heterobifunktionellen Crosslinker-Moleküls, umfassend die folgenden Schritte:
(a) Aufbringen eines Konstrukts aus einem Glykoprotein und einem heterobifunktionellen Crosslinker-Molekül mit Spacer-Bereich, welcher einen Abschnitt zur nicht-kovalenten Anlagerung an eine Oberfläche aufweist, wobei der Spacer-Bereich als Blockcopolymer sowohl einen hydrophilen als auch einen hydrophoben Abschnitt umfasst, und mindestens einer photoreaktiven Gruppe auf eine Oberfläche; und
(b) Immobilisieren des Konstrukts auf der Oberfläche durch Bestrahlen der Kontaktstelle mit Licht einer geeigneten Wellenlänge.

16. Verwendung des Konstrukts gemäß einem der Ansprüche 1 bis 7, der Oberfläche gemäß einem der Ansprüche 8 bis 10, oder der Vorrichtung gemäß Anspruch 11 oder 12 im Rahmen analytischer oder diagnostischer Anwendungen.

## Claims

1. Construct comprising a glycoprotein and a heterobifunctional crosslinker molecule with a spacer region having a section for non-covalent adsorption onto a surface, wherein the spacer region comprises a hydrophilic section as well as a hydrophobic section as a block copolymer, and wherein the crosslinker molecule has at least one photoreactive group for covalent coupling with the surface.

2. Construct according to claim 1, **characterized in that** the section for non-covalent adsorption is hydrophobic or selected from positively or negatively charged hydrophilic sections.

3. Construct according to claim 1 or 2, **characterized in that** the at least one photoreactive group is located at the terminus of the spacer and/or is an integral component of the spacer.

4. Construct according to any of claims 1 to 3, **characterized in that** the at least one photoreactive group is selected from arylazides, benzophenones, benzothiones, anthraquinones, anthrathiones, thymidine, and derivatives thereof.

5. Construct according to any of the preceding claims, **characterized in that** the crosslinker molecule is covalently coupled with the glycoprotein via its carbohydrate component.

6. Construct according to any of the preceding claims, **characterized in that** the glycoprotein is an antibody.

7. Construct according to any of the preceding claims, **characterized in that** the glycoprotein is of recombinant origin.

8. Surface having a construct as defined in any of the claims 1 to 7 covalently immobilized thereon.

9. Surface according to claim 8, comprising a multitude of constructs as defined in any of the claims 1 to 7.

10. Surface according to claim 8 or 9, whose material is selected from the group consisting of glass, quartz glass, quartz, silicon, polymers including PMMA, polystyrene, polyethylene, polypropylene and PVC, membranes including those of nitrocellulose, nylon and microfibers, and paper, as well as mixtures of the same.

11. Analytical or diagnostic apparatus comprising a construct as defined in any of the claims 1 to 7 or a surface as defined in any of the claims 8 to 10.

12. Analytical or diagnostic apparatus according to claim 11, in the form of a biochip, sensor chip, reaction tube or microtiter plate.

13. Method for producing a construct comprising a glycoprotein and a heterobifunctional crosslinker molecule with spacer region having a section for non-covalent adsorption onto a surface, wherein the spacer region comprises a hydrophilic section as well as a hydrophobic section as a block copolymer, and wherein the crosslinker molecule has at least one photoreactive group for covalent coupling with the surface, comprising the following steps:
(a) incubation of the glycoprotein under suitable conditions for creation of aldehyde or thiol groups, respectively;
(b) covalent coupling of the product of step (a) with the crosslinker molecule by means of its amino or maleinimide group(s), respectively; as well as, optionally,
(c) purification and/or isolation of the construct.

14. Method according to claim 13, **characterized in that** the at least one photoreactive group is located at the terminus of the spacer and/or is an integral component of the spacer.

15. Method for covalent immobilization of a glycoprotein on a surface using a heterobifunctional crosslinker molecule comprising the following steps:
(a) applying to a surface a construct of a glycoprotein and a heterobifunctional crosslinker molecule with a spacer region having a section for non-covalent adsorption onto a surface, wherein the spacer region comprises a hydrophilic section as well as a hydrophobic section as a block copolymer, and at least one photoreactive group; and
(b) immobilizing the construct on the surface by irradiation of the contact site with light of a suitable wavelength.

16. Use of the construct as defined in any of claims 1 to 7, of the surface as defined in any of claims 8 to 10, or of the apparatus as defined in claim 11 or 12 in analytical or diagnostic uses.

## Revendications

1. Construction comprenant une glycoprotéine et une molécule de couplage hétéro-bifonctionnelle avec un domaine espaceur (spacer), lequel présente une partie pour l'immobilisation non-covalente sur une surface, où le domaine espaceur contient sous la forme de copolymère à blocs aussi bien une partie hydrophile qu'une partie hydrophobe, et où la molécule de couplage présente au moins un groupe photoréactif pour l'ancrage covalent avec la surface.

2. Construction selon la revendication 1, **caractérisée en ce que** la partie destinée à l'immobilisation non-covalente est choisie parmi des parties hydrophobes ou hydrophiles, chargées positivement ou négativement.

3. Construction selon les revendications 1 ou 2, **caractérisée en ce que** le groupe photoréactif au moins présent dans la construction se trouve à l'extrémité terminale du domaine espaceur et/ou est un élément intégral du domaine espaceur.

4. Construction selon une des revendications 1 à 3, **caractérisée en ce que** le groupe photoréactif au moins présent dans la construction est choisi parmi les arylazides, les benzophénones, les benzothiones, les anthrachinones, les anthrathiones, la thymidine, et les dévirés de ceux-ci.

5. Construction selon une des revendications précédentes, **caractérisée en ce que** la molécule de couplage est liée de manière covalente avec la glycoprotéine par ses éléments glucidiques.

6. Construction selon une des revendications précédentes, **caractérisée en ce que** la glycoprotéine est un anticorps.

7. Construction selon une des revendications précédentes, **caractérisée en ce que** la glycoprotéine est d'origine recombinante.

8. Surface avec une construction selon une des revendication 1 à 7 immobilisée dessus de manière covalente.

9. Surface selon la revendication 8 comprenant une multitude de constructions selon une des revendications 1 à 7.

10. Surface selon la revendication 8 ou 9, dont le matériel est choisi parmi un groupe composé du verre, du verre de quartz, du quartz, du silicium, de polymères, y compris le PMMA, le polystyrol, les polyéthylènes, les polypropylènes et le PVC, des membranes, y compris celles en nitrocellulose, en nylon et en microfibres, et du papier, ainsi que des formes composées de ceux-ci.

11. Dispositif analytique ou diagnostique, comprenant une construction selon une des revendications 1 à 7 ou une surface selon une des revendications 8 à 10.

12. Dispositif analytique ou diagnostique selon la revendication 11 sous forme d'une biopuce, d'une puce sensorielle, d'un tube à essai ou une plaque de microtitration.

13. Procédé pour la production d'une construction comprenant une glycoprotéine et une molécule de couplage hétéro-bifonctionnelle avec un domaine espaceur, lequel présente une partie pour l'immobilisation non-covalente sur une surface, où le domaine espaceur contient sous la forme de copolymère à blocs aussi bien une partie hydrophile qu'une partie hydrophobe, et où la molécule de couplage présente au moins un groupe photoréactif pour l'ancrage covalent avec la surface, comprenant les étapes suivantes:
(a) Incubation de la glycoprotéine sous conditions appropriées pour la création d'un groupe aldéhyde, respectivement thiol;
(b) Liaison covalente du produit de l'étape (a) avec la molécule de couplage par l'intermédiaire de son (ses) groupes amino, respectivement maléinimide; ainsi que, s'il y a lieu
(c) Purification et/ou isolation de la construction.

14. Procédé selon une revendication 13, **caractérisée en ce que** le groupe photoréactif au moins présent dans la construction se trouve à l'extrémité terminale du domaine espaceur et/ou est un élément intégral du domaine espaceur.

15. Procédé pour l'immobilisation covalente d'une glycoprotéine sur une surface en utilisant une molécule de couplage hétéro-bifonctionnelle, comprenant les étapes suivantes:
(a) Dépôt d'une construction comprenant une glycoprotéine et une molécule de couplage hétéro-bifonctionnelle avec un domaine espaceur, lequel présente une partie pour l'immobilisation non-covalente sur une surface, où le domaine espaceur contient sous la forme de copolymère à blocs aussi bien une partie hydrophile qu'une partie hydrophobe, et au moins un groupe photoréactif pour l'ancrage covalent sur une surface; et
(b) Immobilisation de la construction sur la surface par illumination de la position de contact avec de la lumière à une longueur d'onde appropriée.

16. Utilisation d'une construction selon une des revendications 1 à 7, d'une surface selon une des revendications 8 à 10, ou d'un dispositif selon la revendication 11 ou 12 dans le cadre d'une utilisation analytique ou diagnostique.
